# EUROPÄISCHE PATENTSCHRIFT

(11) **EP 2 451 533 B1**
(45) Veröffentlichungstag und Bekanntmachung des Hinweises auf die Patenterteilung: **30.09.2015**
(21) Anmeldenummer: 10720763.1
(22) Anmeldetag: 17.05.2010
(51) Int. Cl.: A61Q 17/04, A61K 8/06, A61K 8/891, B01F 3/08, A61K 8/44, A61K 8/49, A61K 8/35

(54) **VERFAHREN ZUR HERSTELLUNG STABILER EMULSIONEN, INSBESONDERE SONNENSCHUTZFORMULIERUNGEN**
METHOD FOR PRODUCING STABLE EMULSIONS, IN PARTICULAR SUNSCREEN FORMULATIONS
PROCÉDÉ DE PRÉPARATION D'ÉMULSIONS STABLES, NOTAMMENT DE FORMULATIONS DE PROTECTION SOLAIRE

(30) Priorität: 09.07.2009 DE 102009027584
(43) Veröffentlichungstag der Anmeldung: 16.05.2012
(73) Patentinhaber: Henkel AG & Co. KGaA, 40589 Düsseldorf (DE)
(72) Erfinder: YÜCEL, Sevda, 41470 Neuss (DE); WALDMANN-LAUE, Marianne, 40789 Monheim (DE); DICKHOF, Susanne, 41748 Viersen (DE); JANßEN, Frank, 41470 Neuss (DE)
(86) Internationale Anmeldenummer: PCT/EP2010/056723
(87) Internationale Veröffentlichungsnummer: WO 2011/003655

(56) Entgegenhaltungen:
- DE-A1- 10 217 255
- GB-A- 1 247 009
- US-A- 2 983 615
- US-A- 3 497 535
- US-A- 4 631 136
- E. S. SCHERWIN: "Oxidation and Antioxidants in Fat and Oil Processing", JOURNAL OF THE AMERICAN OIL CHEMIST'S SOCIETY, Bd. 55, November 1978 (1978-11), Seiten 809-814, XP007918718,

## Beschreibung

Die Erfindung betrifft insbesondere das Gebiet der Herstellung stabiler Öl-in-Wasser-Emulsionen. Die Erfindung betrifft insbesondere ein Verfahren zur Herstellung von stabilen Emulsionen, die als kosmetische Sonnenschutzmittel eingesetzt werden.

Emulsionen können breit als metastabile kolloidale Dispersionen von Flüssigkeitstropfen in einer anderen flüssigen Phase definiert werden. Typischerweise sind Emulsionen disperse Systeme, die zumindest zwei Flüssigkeiten umfassen, die tatsächlich ineinander unlöslich sind. Neben den zumindest zwei Bestandteilen der flüssigen Phasen und wahlweise festen Teilchen und/oder einem oder mehreren Emulgatoren erfordert die Bildung einer Emulsion Energie und tritt bei Bedingungen auf, die von einem Gleichgewicht weit entfernt sind. Im Allgemeinen umfasst die Bildung einer Emulsion die zwei folgenden Schritte:
•- in einem initialen Schritt werden zumindest zwei Bestandteile vorgemischt, wobei die zumindest zwei Bestandteile die ursprünglich nicht-mischbare flüssige Phase waren, wobei das Vormischen vorzugsweise in Gegenwart einer geeigneten Menge eines oder mehrerer Emulgatoren stattfindet, um Tröpfchen einer dispergierten Flüssigkeitsphase in einer anderen kontinuierlichen Flüssigkeitsphase zu erzeugen;
•- danach werden die sich aus dem initialen Schritt ergebenden Tröpfchen durch Scherkräfte oder durch lokale Druckdifferenzen zerrissen, d. h. durch Trägheitskräfte, wodurch sich eine stabilere Emulsion üblicherweise kleinerer Tröpfchen ergibt.

Gegenwärtig werden unterschiedliche Arten von mechanischen Emulgier-Verfahren in der Herstellung vor. fein dispergierten Emulsionen verwendet, wobei jedes Verfahren eine spezielle Ausstattung erfordert. Innerhalb dieser Emulsifikationssysteme können vier Hauptkategorien unterschieden werden:
•- das Zerreißen von Tröpfchen in einem stark scherenden Rotor-Stator-System,
•- das Zerreißen von Tröpfchen durch Ultraschall,
•- das Zerreißen von Tröpfchen in Hochdrucksystemen, und
•- die Tröpfchenbildung an Mikroporen (unter Verwendung mikroporöser Membranen oder von Mikrokanälen).

Emulsionen können entweder direkt als Konsumentenprodukte oder als Zwischenprodukte zur Verwendung eines breiten Bereichs industrieller Anwendungen hergestellt werden, einschließlich in einer bei weitem nicht erschöpfenden Liste, Nahrungsmitteln, Farben, Kosmetika, pharmazeutischen Produkten, Explosivstoffen, Raketentreibstoff, Gleitmitteln, Schaumkontrollmitteln etc. Die meisten industriellen Anwendungen oder Konsumentenprodukte erfordern, dass die Emulsionen eine maximale Lagerstabilität aufweisen. Die Lagerstabilität betrifft die Zeitspanne, während der die Emulsion aufrechterhalten werden kann, bevor sie sich wiederum in unterschiedliche Phasen auftrennt. Die Mechanismen, die im Verfahren zum Brechen einer Emulsion identifiziert werden können, schließen das so genannte Ostwald-Ripening, Aufrahmen, Aggregation, Koaleszenz und Teilkoaleszenz ein. Das Verfahren zum Brechen einer Emulsion kann beeinflusst oder überwacht werden, und deswegen kann die Lagerstabilität auf den folgenden Wegen kontrolliert oder erhöht werden: Unter Verwendung mechanischer Vorrichtungen zur Kontrolle der Größe der dispergierten Tropfen und/oder durch Zusetzen von stabilisierenden chemischen Zusatzstoffen oder Emulgatoren, um die Emulsion in Dispersion zu halten.

Wenn die zumindest zwei Flüssige-Phasen-Bestandteile einer Emulsion zusammen vermischt werden, ist das initiale Ergebnis eine grob disperse Rohemulsion. Durch Zusetzen mechanischer Energie werden die großen Tropfen der Rohemulsion aufgebrochen und die erwünschte Feinemulsion wird gebildet. Die kleinste erreichbare Tröpfchengröße im letzten Schritt des Emulgierverfahrens hängt nicht nur vom jeweiligen Energieaufwand in den Emulgiergeräten ab, sondern kann auch entscheidend durch die Art und Konzentration der Emulgierhilfsstoffe beeinflusst werden. Um beispielsweise ultradünne Emulsionen zu erzeugen, ist es essentiell, dass eine Grenzfläche, die mechanisch gebildet werden, sehr schnell durch den Emulgator besetzt werden, um eine Koaleszenz der Tröpfchen zu vermeiden.

Die durchschnittliche Größe der Tröpfchen der dispersen Phase kann gemäß des Prinzips der quasi-elastischen dynamischen Lichtstreuung bestimmt werden, beispielsweise durch Verwendung eines Coulter N4+ Teilchenanalysegerätes, das kommerziell von Coulter Scientific Instruments erhältlich ist.

An kritische Emulsionen, insbesondere an Sonnenschutzmittel in Emulsionsform, werden weitere Anforderungen gestellt. So muß eine kosmetische Emulsion langzeitstabil sein und darf sich auch nach Öffnen der Verpackung nicht in ihrer Lagerstabilität beeinflussen lassen. Zusätzlich ist die Farbe und Farbstabilität der Emulsion von entscheidender Bedeutung. Reinweiße Produkte werden vom Verbraucher deutlich positiver bewertet, Produkte, die Farbveränderungen unterliegen, werden als nicht akzeptabel wahrgenommen.

Kosmetische Emulsionen, die Komplexbildner enthalten, neigen gelegentlich zu Verfärbungen bei längerer Lagerzeit. Dieses Problem tritt insbesondere dann auf, wenn gleichzeitig UV-Filtersubstanzen in den Emulsionen enthalten sind.

Es wurde nun gefunden, dass die Einarbeitung der Komplexbildner einen entscheidenden Einfluss auf die Farbstabilität der Emulsionen hat.

US 2983615 beschäftigt sich mit der Lagerstabilität von Margarine mittels Zitronensäure und EDTA. US 4631136 beschäftigt sich damit, Bohrflüssigkeiten auf Basis von natürlichen Ölen gegenüber der hohen Scher- und Temperaturbelastung, die bei der Gewinnung von Erdöl und Erdgas auftritt und zur Oxidation und Polymerisation der ungesättigten Fettsäuren in den natürlichen Ölen führt, mittels Zitronensäure, Ascorbinsäure oder Phosphorsäure in der Ölphase zu stabilisieren. US 3497535 offenbart Fette und Öle, die durch EDTA-Esterderivate stabilisiert werden. GB 1247009 offenbart Seifenstücke, enthaltend eine Ölphase, aus Talg, Kokosöl und freier Fettsäure, die durch einen Komplexbildner gegen Verfärbungen und schlechte Gerüche stabilisiert sind.

In der deutschen Offenlegungsschrift DE 102 17 255 A1 werden Sonnenschutzemulsionen beschreiben, die neben UV-Schutzfiltern den Komplexbildner EDTA-Tetranatriumsalz enthalten.

Gegenstand der vorliegenden Erfindung ist ein Verfahren zur Herstellung von Emulsionen, welche Komplexbildner enthalten, bei dem die Schritte:
a) Bereitstellen einer Ölphase;
b) Einarbeitung mindestens eines Komplexbildners in die Ölphase, wobei der Komplexbildner in fester Form vorliegt;
c) Vermischen mit einer Wasserphase;
d) Emulgieren
nacheinander durchgeführt werden, wobei die Ölphase mindestens einen öllöslichen UV-Filter, umfassend mindestens ein Alkyl- und/oder Alkoxy-substituiertes Dibenzovlmethanderivat und/oder Polysilicone-15, enthält und der Komplexbildner ausgewählt ist aus Ethvlendiamintetraessigsäure (EDTA) und ihren Salzen, insbesondere Calcium-Dinatrium-EDTA, Nitrilotriessigsäure (NTA), Zitronensäure und ihren Salzen Natriumcitrat, Kaliumcitrat und Calciumcitrat, Phosphaten, Oxydisuccinaten, Ethylendiamin-N,N'-disuccinat (EDDS), bevorzugt in Form seiner Natrium- oder Magnesiumsalze, Glycerindisuccinaten, Glycerintrisuccinaten, Iminodisuccinat Tetranatriumsalz, Gluconsäure, insbesondere in Form des leicht kristallisierenden Glucono-6-lactons, Isoascorbinsäure, Natriumisoascorbat und Weinsäure und ihren Salzen.

In bevorzugten erfindungsgemäßen Verfahren beträgt der Anteil der Ölphase 2 bis 50 Gew.-%, vorzugsweise 3 bis 45 Gew.-%, weiter bevorzugt 4 bis 40 Gew.-% und insbesondere 5 bis 30 Gew.-%, bezogen auf das Gesamtgewicht der Emulsion.

Die Ölphase kann erhitzt werden, um den späteren Emulgiervorgang zu erleichtern. Hier sind bevorzugte erfindungsgemäße Verfahren dadurch gekennzeichnet, dass die Ölphase eine Temperatur von 60 bis 95°C, vorzugsweise von 65 bis 90°C, besonders bevorzugt von 70 bis 90°C und insbesondere von 75 bis 85°C, aufweist.

Das erfindungsgemäße Verfahren ist aber auch hervorragend für die Kaltemulgierung geeignet, also für Emulgierverfahren, bei denen die Öl- und die Wasserphase ohne vorherige Erwärmung miteinander vermischt werden.

Weitere bevorzugte erfindungsgemäße Verfahren sind daher dadurch gekennzeichnet, dass die Ölphase eine Temperatur von 15 bis 30°C, vorzugsweise von 18 bis 28°C, besonders bevorzugt von 20 bis 25°C, aufweist.

Weitere bevorzugte erfindungsgemäße Verfahren sind dadurch gekennzeichnet, dass die Ölphase eine Temperatur von 32 bis 55°C, vorzugsweise von 35 bis 50°C, besonders bevorzugt von 40 bis 45°C, aufweist.

Erfindungsgemäß wird mindestens ein Komplexbildner in fester Form in die Ölphase eingearbeitet, bevor die Wasserphase mit der Ölphase vermischt wird.

Komplexbildner sind wasserlöslich und werden daher üblicherweise der wässrigen Phase der Emulsion vor dem Emulgiervorgang zugegeben. Gelegentlich wird eine wässrige Lösung des Komplexbildners in der fertigen Emulsion nachemulgiert. Erfindungsgemäß wird der Komplexbildner (bzw. werden mehrere Komplexbildner) in fester Form in die Ölphase eingearbeitet, bevor die Wasserphase zugegeben und der Emulgiervorgang durchgeführt wird. "Feste Form" bezieht sich dabei auf den Aggregatzustand bei 20°C.

Bevorzugte Emulsionen enthalten den bzw. die Komplexbildner in bestimmten Mengen. Hier sind erfindungsgemäße Verfahren bevorzugt, bei denen der Anteil der Komplexbildner 0,01 bis 2,5 Gew.-%, vorzugsweise 0,025 bis 0,5 Gew.-%, weiter bevorzugt 0,05 bis 0,25 Gew.-% und insbesondere 0,06 bis 0,1 Gew.-%, bezogen auf das Gesamtgewicht der Emulsion, beträgt.

Je nach Anteil der Ölphase an der Emulsion ergeben sich damit höhere Gew.-%-Anteile der Komplexbildner in der Ölphase.

Neben dem bzw. den Komplexbildner(n) kann die Ölphase weitere Inhaltsstoffe der Emulsion enthalten. Bewährt haben sich beispielsweise öllösliche Emulgatoren, Konservierungsstoffe, Farb- und Duftstoffe usw. Besonders bevorzugt ist der Einsatz öllöslicher UV-Filtersubstanzen in der Ölphase, siehe weiter unten.

Zusätzlich zur Ölphase wird eine wässrige Phase (Wasserphase) bereitgestellt, die ebenfalls weitere Inhaltsstoffe enthalten kann. Auch die Wasserphase kann zur Verbesserung des Emulgiervorgangs erwärmt werden, bevorzugte erfindungsgemäße Verfahren sind dadurch gekennzeichnet, dass die Wasserphase eine Temperatur von 60 bis 95°C, vorzugsweise von 65 bis 90°C, besonders bevorzugt von 70 bis 90°C und insbesondere von 75 bis 85°C aufweist.

Das erfindungsgemäße Verfahren ist, wie verstehend bereits erwähnt, aber auch hervorragend für die Kaltemulgierung geeignet, also für Emulgierverfahren, bei denen die Öl- und die Wasserphase ohne vorherige Erwärmung miteinander vermischt werden.

Weitere bevorzugte erfindungsgemäße Verfahren sind daher dadurch gekennzeichnet, dass die Wasserphase eine Temperatur von 15 bis 30°C, vorzugsweise von 18 bis 28°C, besonders bevorzugt von 20 bis 25°C, aufweist.

Weitere bevorzugte erfindungsgemäße Verfahren sind dadurch gekennzeichnet, dass die Wasserphase eine Temperatur von 32 bis 55°C, vorzugsweise von 35 bis 50°C, besonders bevorzugt von 40 bis 45°C, aufweist.

Ölphase und Wasserphase werden miteinander vermischt. Dabei kann die Wasserphase zur Öl-phase gegeben werden, es ist aber auch möglich, die Ölphase in die Wasserphase einzuarbeiten. Welche der Phasen vorgelegt und welche zugegeben wird, hängt dabei auch davon ab, ob das Endprodukt eine Öl-in-Wasser-Emulsion oder eine Wasser-in-Öl-Emulsion sein soll.

Das erfindungsgemäße Verfahren ist darüber hinaus auch zur Herstellung multipler Emulsionen, insbesondere von Wasser-in-Öl-in-Wasser-Emulsionen und Öl-in-Wasser-in-Öl-Emulsionen, geeignet.

Je nach gewünschter Viskosität der Emulsion können dabei die Mengenanteile weiterer Inhaltsstoffe und die zur Emulgierung eingesetzten Apparate breit variiert werden.

Wie bereits erwähnt, sind Sonnenschutzemulsionen besondere Ausführungsformen der vorliegenden Erfindung, da die zusätzliche Anwesenheit von UV-Filtersubstanzen die weiter oben geschilderten Probleme bei nicht-erfindungsgemäßer Vorgehensweise verstärkt. Durch das erfindungsgemäße Verfahren lassen sich auch Sonnenschutzemulsionen lager- und farbstabil herstellen. Erfindungsgemäße Verfahren sind dadurch gekennzeichnet, dass die Ölphase - bezogen auf das Gesamtgewicht der Emulsion - 0,5 bis 20 Gew.-%, vorzugsweise 0,75 bis 15 Gew.-%, weiter bevorzugt 1 bis 15 Gew.-%, noch weiter bevorzugt 1,5 bis 10 Gew.-% und insbesondere 3 bis 8 Gew.-% UV-Filtersubstanzen enthält, wobei die Ölphase mindestens einen öllöslichen UV-Filter, umfassend mindestens ein Alkyl- und/oder Alkoxy-substituiertes Dibenzoylmethanderivat und/oder Polysilicone-15, enthält.

Erfindungsgemäß bevorzugte Alkyl- und/oder Alkoxy-substituierte Dibenzoylmethanderivate sind dadurch gekennzeichnet, dass einer der Phenylreste mit mindestens einer Alkylgruppe, ausgewählt aus Methyl, Ethyl, n-Propyl, Isopropyl, n-Butyl, sec-Butyl, tert.-Butyl, n-Pentyl, Isopentyl, Neopentyl, 2-Ethylhexyl, und der andere Phenylrest mit mindestens einer Alkoxy-Gruppe, ausgewählt aus Methoxy, Ethoxy, n-Propoxy, Isopropoxy, n-Butoxy, sec-Butoxy, tert.Butoxy, n-Pentoxy, Isopentoxy, Neopentoxyl, 2-Ethylhexoxy, substituiert ist. Besonders bevorzugte Substituenten sind Isopropyl, tert.-Butyl und Methoxy. Erfindungsgemäß bevorzugte Alkyl- und/oder Alkoxy-substituierte Dibenzoylmethanderivate sind ausgewählt aus 2-Methyldibenzoylmethan, 4-Methyldibenzoylmethan, 4-Isopropyldibenzoylmethan, 4-tert-Butyldibenzoylmethan, 2,4-Dimethyldibenzoylmethan, 2,5-Dimethyldibenzoylmethan, 4,4'-Diisopropyldibenzoylmethan, 4,4'-Dimethoxydibenzoylmethan, 4-tert-Butyl-4'-Methoxydibenzoylmethan, 2-Methyl-5-isopropyl-4'-methoxydibenzoylmethan, 2-Methyl-5-tert-butyl-4'-methoxydibenzoylmethan, 2,4-Dimethyl-4'-methoxydibenzoylmethan, 2,6-Dimethyl-4-tert-butyl-4'-methoxydibenzoylmethan. Besonders bevorzugt ist 4-tert-Butyl-4'-Methoxydibenzoylmethan mit der INCI-Bezeichnung Butyl Methoxydibenzoylmethane (auch als 1-(4'-tert-Butylphenyl)-3-(4'-methoxyphenyl)propan-1,3-dion bezeichnet), ein öllöslicher organischer Lichtschutzfilter, der z. B. als PARSOL^{®} 1789 von DSM oder als Eusolex^{®} 9020 von Merck KGaA erhältlich ist.

Die Substanz mit der INCI-Bezeichnung Polysilicone-15 wird auch als 3-(4-(2,2-Bis-Ethoxycarbonylvinyl)-phenoxy)propenyl)-methoxysiloxan/Dimethylsiloxan-Copolymer mit der Parsol^{®} SLX), Dimethicodiethylbenzalmalonat, Diethylbenzylidene Malonate Dimethicone oder Diethylmalonylbenzylidene Oxypropene Dimethicone bezeichnet und ist unter dem Handelsnamen Parsol^{®} SLX (INCI-Bezeichnung Dimethicodiethylbenzal malonate (CAS-Nr. 207574-74-1)) von DSM erhältlich.

Auch die Wasserphase kann UV-Filtersubstanzen enthalten. Hier sind erfindungsgemäße Verfahren bevorzugt, bei denen die Wasserphase - bezogen auf das Gesamtgewicht der Emulsion - 0,5 bis 10 Gew.-%, vorzugsweise 0,75 bis 7,5 Gew.-%, weiter bevorzugt 1 bis 5 Gew.-%, noch weiter bevorzugt 1,5 bis 4 Gew.-% und insbesondere 2 bis 3 Gew.-% UV-Filtersubstanzen enthält.

Ganz besonders bevorzugte Verfahren sind dadurch gekennzeichnet, dass die Wasserphase mindestens einen wasserlöslichen UV-Filter, umfassend ein Derivat der Benzimidazolsulfonsäure, enthält.

Bevorzugte UV-Filtersubstanz(en) für die Wasserphase ist bzw. sind die Phenylen-1,4-bis-(2-benzimidazyl)-3,3'-5,5'-tetrasulfonsäure (UV-A) und ihre Salze, insbesondere die Alkali-, Erdalkali-, Ammonium-, Alkylammonium-, Alkanolammonium- und Glucammoniumsalze, bevorzugt die entsprechenden Natrium-, Kalium-, Trialkylammonium- oder Triethanolamin-Salze, insbesondere das Phenylen-1,4-bis-(2-benzimidazyl)-3,3'-5,5'-tetrasulfonsäure-bis-natriumsalz mit der INCI-Bezeichnung "Disodium Phenyl Dibenzimidazole Tetrasulfonate" (CAS-Nr.: 180898-37-7), das beispielsweise unter der Handelsbezeichnung Neo Heliopan AP von Symrise erhältlich ist. Weitere bevorzugte UV-Filtersubstanz(en) zum Einsatz in der Wasserphase ist bzw. sind die 2-Phenylbenzimidazol-5-sulfonsäure (UV-B) und ihre Salze, insbesondere die Alkali-, Erdalkali-, Ammonium-, Alkylammonium-, Alkanolammonium- und Glucammoniumsalze, bevorzugt die entsprechenden Natrium-, Kalium-, Trialkylammonium- oder Triethanolamin-Salze, insbesondere aber die 2-Phenylbenzimidazol-5-sulfonsäure selbst mit der INCI-Bezeichnung "Phenylbenzimidazole sulfonic acid" (CAS.-Nr. 27503-81-7), die beispielsweise unter den Handelsnamen Neo Heliopan Hydro von Symrise oder Eusolex 232 von Merck KGaA erhältlich ist.

In einer erfindungsgemäß besonders bevorzugten Ausführungsform sind die Salze der 2-Phenylbenzimidazol-5-sulfonsäure ausgewählt aus den Salzen dieser Säure mit basischen Aminosäuren, insbesondere mit Lysin, Arginin und/oder Histidin, wobei Arginin besonders bevorzugt ist.

In einer weiteren erfindungsgemäß besonders bevorzugten Ausführungsform sind die Salze der Phenylen-1,4-bis-(2-benzimidazyl)-3,3'-5,5'-tetrasulfonsäure ausgewählt aus den Salzen dieser Säure mit basischen Aminosäuren, insbesondere mit Ornithin, Lysin, Arginin und/oder Histidin, wobei Arginin besonders bevorzugt ist.

Erfindungsgemäß besonders bevorzugte Emulsionen enthalten Lichtschutzfilter-Kombinationen aus 4-tert-Butyl-4'-Methoxydibenzoylmethan, Polysilicone-15 und 2-Phenylbenzimidazol-5-sulfonsäure. Weitere erfindungsgemäß besonders bevorzugte Lichtschutzfilter-Kombinationen sind 4-tert-Butyl-4'-Methoxydibenzoylmethan, Polysilicone-15 und Dinatriumphenylbenzimidazoltetrasulfonat. Weitere erfindungsgemäß besonders bevorzugte Lichtschutzfilter-Kombinationen umfassen 4-tert-Butyl-4'-Methoxydibenzoylmethan, Polysilicone-15 und ein 2-Phenylbenzimidazol-5-sulfonsäure-Arginin-Salz.

Bevorzugte erfindungsgemäß hergestellte Emulsionen sind dadurch gekennzeichnet, dass die UV-Filter aus der Gruppe der Alkyl- und/oder Alkoxy-substituierten Dibenzoylmethanderivate (a) und Polysilicone-15 (b) und aus der Gruppe der Derivate der Benzimidazolsulfonsäure (c) in einem Gewichtsverhältnis zueinander von a) : b) : c) wie (0,8 - 1,5) : (0,8 - 1,5) : (0,8 - 1,5), bevorzugt wie (0,9 - 1,2) : (0,9 - 1,2) : (0,9 - 1,2), besonders bevorzugt wie (1 - 1,1): (1 - 1,1) : (1 - 1,1), enthalten sind.

Um den Lichtschutzfaktor weiter zu erhöhen, können die erfindungsgemäß hergestellten Emulsionen bevorzugt mindestens eine weitere organische und/oder anorganische UV-Filtersubstanz enthalten. Diese können der fertigen Emulsion zugesetzt werden, es ist aber auch möglich, die öllöslichen Substanzen vorher in die Ölphase einzubringen und analog die wasserlöslichen in die Wasserphase.

Weitere bevorzugte UV-Filtersubstanzen sind im Folgenden genannt.

### Triazinderivate

### UV-Filtersubstanzen auf Basis von Triazinderivaten, die das nachfolgende Strukturmotiv

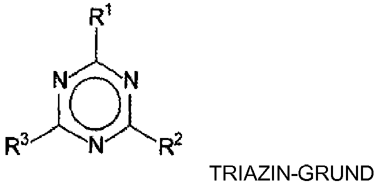

aufweisen, sind im Stand der Technik bekannt, beispielsweise aus EP 775698, EP 878469 und EP 1027881.

Hinsichtlich der C₃-Achse des Triazin-Grundkörpers dieser Verbindungen sind sowohl symmetrische Substitution als auch unsymmetrische Substitution denkbar.

In diesem Sinne symmetrisch substituierte s-Triazine weisen drei gleiche Substituenten R¹, R² und R³ auf, während unsymmetrisch substituierte s-Triazinderivate demzufolge unterschiedliche Substituenten aufweisen, wodurch die C₃-Symmetrie zerstört wird. Im Sinne der vorliegenden Erfindung wird als "unsymmetrisch" stets unsymmetrisch hinsichtlich der C₃-Achse des Triazingrundkörpers verstanden, es sei denn, etwas anderes wäre ausdrücklich erwähnt.

Hinsichtlich der C₃-Achse des Triazin-Grundkörpers symmetrische Triazinderivate sind bevorzugt solche der allgemeinen Formel TRIAZIN-GRUND mit R¹ = R² = R³ = -NH-Phe-COOR, mit Phe = Phenyl-Rest, bei dem die Substituenten -NH und -COOR in para-Position zueinander stehen. Besonders bevorzugte derartige symmetrische Triazinderivate sind 4,4',4"-(1,3,5-Triazin-2,4,6-triyltriimino)-tris-benzoesäure-tris(alkylester). Ein besonders bevorzugter derartiger Ester ist 4,4',4"-(1,3,5-Triazin-2,4,6-triyltriimino)-tris-benzoesäure-tris(2-ethylhexylester) [INCl: Ethylhexyl Triazone, vormals Octyl Triazone], das als Einzelsubstanz von BASF unter dem Handelsnamen UVINUL® T 150 vertrieben wird, aber auch in diversen kommerziellen UV-Filtermischungen erhältlich ist. Erfindungsgemäß bevorzugte unsymmetrische Triazinderivate sind beispielsweise solche, die in EP 775698 offenbart sind: 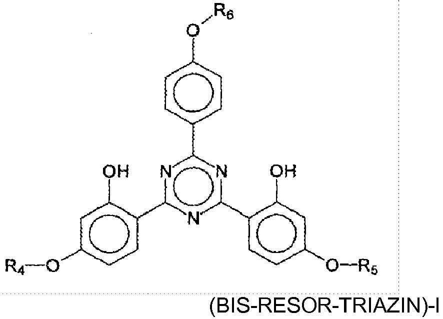 und/oder

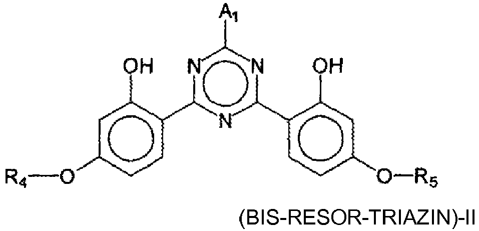

Alle in EP 775698 erwähnten so genannten Bis-Resorcinyltriazine, seien sie durch generische oder durch konkrete Formeln offenbart, sind vorteilhaft im Sinne der vorliegenden Erfindung.

Ganz besonders bevorzugt werden R₄ und R₅ aus der Gruppe der verzweigten und unverzweigten Alkylgruppen von 1 bis 18 Kohlenstoffatomen gewählt. Auch können die Alkylgruppen wiederum vorteilhaft mit Silyloxygruppen substituiert sein.

A₁ stellt bevorzugt einen substituierten homo- oder heterocyclischen aromatischen Fünfring oder Sechsring dar.

Ganz besonders bevorzugte optionale UV-Filtersubstanzen sind ausgewählt aus unsymmetrisch substituierten s-Triazin-Verbindungen der allgemeinen Formel (BIS-RESOR-TRIAZIN)-I, in der R₆ ein Wasserstoffatom oder eine verzweigte oder unverzweigte Alkylgruppe mit 1 bis 10 Kohlenstoffatomen darstellt. Eine besonders bevorzugte Verbindung der allgemeinen Formel (BIS-RESOR-TRIAZIN)-I, in der R₄ und R₅ jeweils eine 2-Ethylhexyl-Gruppe und R₆ eine Methylgruppe darstellen, ist 2,4-Bis-{[4-(2-ethyl-hexyloxy)-2-hydroxy]-phenyl}-6-(4-methoxyphenyl)-1,3,5-triazin (INCI: Bis-Ethylhexyloxyphenol Methoxyphenyl Triazine), unter dem Handelsnamen Tinosorb^{®} S von CIBA erhältlich.

Eine weitere, erfindungsgemäß bevorzugte optionale unsymmetrisch substituierte s-Triazin-Verbindung ist eine Verbindung mit der INCI-Bezeichnung Diethylhexyl Butamido Triazone, mit der allgemeinen Formel TRIAZIN-GRUND mit R¹ = R² -NH-Phe-COOR, mit Phe = Phenyl-Rest, bei dem die Substituenten -NH und -COOR in para-Position zueinander stehen, R = 2-Ethylhexyl und R³ = -NH-Phe-CONH-tert-Butyl, mit Phe = Phenyl-Rest, bei dem die Substituenten -NH und -CONH-tert.-Butyl in para-Position zueinander stehen. Diese UV-Filtersubstanz, ein effektiver UV-A-Filter, ist unter der Handelsbezeichnung UVASORB HEB bei Sigma 3V erhältlich ist.

Weitere, erfindungsgemäß bevorzugte optionale UV-Filtersubstanzen auf Basis von s-Triazin-Verbindungen sind:
- 2,4,6-Tris([1,1'-Biphenyl]-4-yl)-1,3,5-Triazine, INCI: Tris-Biphenyl Triazine, erhältlich unter dem Handelsnamen Tinosorb A2B von CIBA,
- 2,4-bis-[5-1(di-methylpropyl)benzoxazol-2-yl-(4-phenyl)-imino]-6-(2-ethylhexyl)-imino-1,3,5-triazin (CAS Nr. 288254-16-0, Uvasorb^{®} K2A von 3V Sigma, INCI: Ethylhexyl Bis-Isopentylbenzoxazolylphenyl Melamine),
- 2,4-Bis-{[4-(3-sulfonato)-2-hydroxy-propyloxy)-2-hydroxy]-phenyl}-6-(4-methoxyphenyl)-1,3,5-triazin-Natriumsalz,
- 2,4-Bis-{[4-(3-(2-propyloxy)-2-hydroxy-propyloxy)-2-hydroxy]-phenyl}-6-(4-methoxyphenyl)-1,3,5-triazin,
- 2,4-Bis-{[4-(2-ethyl-hexyloxy)-2-hydroxy]-phenyl}-6-[4-(2-methoxyethyl-carboxyl)-phenylamino]-1,3,5-triazin,
- 2,4-Bis-{[4-(3-(2-propyloxy)-2-hydroxy-propyloxy)-2-hydroxy]-phenyl}-6-[4-(ethylcarboxyl)-phenylamino]-1, 3,5-triazin,
- 2,4-Bis-{[4-(2-ethyl-hexyloxy)-2-hydroxy]-phenyl}-6-(1-methyl-pyrrol-2-yl)-1,3,5-triazin,
- 2,4-Bis-{[4-tris(trimethylsiloxy-silylpropyloxy)-2-hydroxy]-phenyl}-6-(4-methoxyphenyl)-1,3,5-triazin,
- 2,4-Bis-([4-(2-methylpropenyloxy)-2-hydroxy]-phenyl}-6-(4-methoxyphenyl)-1,3 ,5-triazin und
- 2,4-Bis-{[4-(1',1',1',3',5',5',5'-Heptamethylsiloxy-2-methyl-propyloxy)-2-hydroxy]-phenyl}-6-(4-methoxyphenyl)-1,3,5-triazin,
- 2-[4,6-Bis(2,4-dimethylphenyl)-1,3,5-triazin-2-yl]-5-(ethylhexyloxy)phenol.

Die s-Triazinderivate werden bevorzugt in die Ölphase der erfindungsgemäßen kosmetischen oder dermatologischen Zusammensetzungen eingearbeitet.

### Benzotriazole

### Eine weitere bevorzugte optionale UV-Filtersubstanz ist 2,2'-Methylen-bis-(6-(2H-benzotriazol-2-yl)-4-(1,1,3,3-tetramethylbutyl)-phenol) (INCI: Methylene Bis-Benzotriazolyl Tetramethylbutylphenol, MBBT) mit folgender Strukturformel

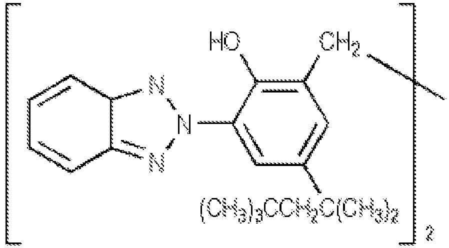

, unter der Handelsbezeichnung Tinosorb^{®} M von CIBA erhältlich. Hierbei handelt es sich um einen so genannten Breitbandfilter, der sowohl UV-A- als auch UV-B-Strahlung absorbiert.

Eine weitere bevorzugte optionale Breitband-UV-Filtersubstanz ist 2-(2H-Benzotriazol-2-yl)-4-methyl-6-[2-methyl-3-[1,3,3,3-tetramethyl-1-((trimethylsilyl)oxy]disiloxanyl]propyl]-phenol (CAS-Nr.: 155633-54-8) mit der INCI-Bezeichnung Drometrizole Trisiloxane.

Weitere bevorzugte optionale Benzotriazol-Filter sind 2,2'-Methyl-bis-[6(2H-benzotriazol-2-yl)-4-(methyl)phenol] (MIXXIM BB/200 der Firma Fairmount Chemical), 2-(2'-Hydroxy-3',5'-di-t-amyl-phenyl)benzotriazol (CAS- Nr.: 025973-551), 2-(2'-Hydroxy-5'-octylphenyl)-benzotriazol (CAS-Nr. 003147-75-9), 2-(2'-Hydroxy-5'-methylphenyl)benzotriazol (CAS-Nr. 2440-22-4) und 2-(2H-benzotriazol-2-yl)-4-methyl-6-[2-methyl-3-[1,3,3,3-tetramethyl-1-((trimethylsilyl)oxy]disiloxanyl)propyl]-phenol (CAS-Nr.: 155633-54-8) mit der INCI-Bezeichnung Drometrizole Trisiloxane.

Weitere bevorzugte UV-Filtersubstanzen, die neben denen der Komponenten (a), (b) und (c) in den erfindungsgemäßen Zusammensetzungen eingesetzt werden, sind im Folgenden genannt:
- Derivate des Camphers, insbesondere 3-Benzylidencampher-Derivate, die keine ionisierbaren funktionellen Gruppen im Molekül aufweisen können, bevorzugt 3-(4'-Methylbenzyliden)-D,L-campher [INCI: 4-Methylbenzylidene Camphor], das von Merck unter der Warenbezeichnung Eusolex 6300 vertrieben wird;
- Derivate des Camphers, die ionisierbare funktionelle Gruppen im Molekül aufweisen können, bevorzugt Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze; das 1,4-di(2-oxo-10-Sulfo-3-bornylidenmethyl)-Benzol und dessen Salze (besonders die entsprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird, Sulfonsäurederivate des 3-Benzylidencamphers, wie z. B. 4-(2-Oxo-3-bornylidenmethyl)benzolsulfonsäure und 2-Methyl-5-(2-oxo-3-bornyliden)sulfonsäure und deren Salze mit der INCI-Bezeichnung Terephthalylidene Dicamphor Sulfonic Acid (CAS.-Nr.: 92761-26-7, als Mexoryl SX von der Firma Chimex erhältlich).
- 4-Aminobenzoesäure-Derivate, bevorzugt 4-(Dimethylamino)-benzoesäure(2-ethylhexyl) ester, 4-(Dimethylamino)benzoësäureamylester;
- 2-Aminobenzoesäure-Derivate
- Ester der Zimtsäure, bevorzugt 4-Methoxyzimtsäure(2-ethylhexyl)ester, 4-Methoxyzimtsäurepropylester, 4-Methoxyzimtsäureisopentylester; und 2-Cyano-3,3-phenylzimtsäure-2-ethylhexylester (= Ethylhexyl-2-cyano-3,3-diphenylacrylat (Octocrylene)),
- Ester der Salicylsäure, bevorzugt Salicylsäure(2-ethylhexyl)ester (2-Ethylhexylsalicylat (=Octylsalicylat)), Salicylsäure(4-isopropylbenzyl)ester (4-Isopropylbenzylsalicylat), Salicylsäurehomomenthylester (Homomenthylsalicylat, Homosalate).
- Derivate des Benzophenons, die keine ionisierbaren funktionellen Gruppen im Molekül aufweisen, bevorzugt 2-Hydroxy-4-methoxy-4'-methylbenzophenon, 2,2'-Dihydroxy-4-methoxybenzophenon, 2-Hydroxy-4-methoxybenzophenon, 2-Hydroxy-4-methoxy-4'-methylbenzophenon, 2,2'-Dihydroxy-4-methoxybenzophenon; 2-(4'-Diethylamino-2'-hydroxybenzoyl)-benzoesäurehexylester (auch: Aminobenzophenon, unter der Bezeichnung Uvinul A Plus von BASF erhältlich),
- Derivate des Benzophenons, die ionisierbare funktionelle Gruppen im Molekül aufweisen, bevorzugt Sulfonsäurederivate von Benzophenonen, besonders bevorzugt 2-Hydroxy-4-methoxybenzophenon-5-sulfonsäure und ihre Salze,
- Ester der Benzalmalonsäure, bevorzugt 4-Methoxybenzalmalonsäuredi(2-ethylhexyl)ester,
- an Polymere gebundene UV-Filter, die von Komponente (b) verschieden sind;
- Derivate von Benzoxazol, bevorzugt 2,2'(Naphthalene-1,4-Diyl)bis(benzoxazole) (INCI: Dibenzoxazoyl Naphthalene) und 2,4-bis-[5-1(di-methylpropyl)benzoxazol-2-yl-(4-phenyl)-imino]-6-(2-ethylhexyl)-imino-1,3,5-triazin (Uvasorb^{®} K2A von 3V Sigma, INCI: Ethylhexyl Bis-Isopentylbenzoxazolylphenyl Melamine).

Die Benzoxazol-Derivate liegen bevorzugt in gelöster Form in den erfindungsgemäßen kosmetischen Zusammensetzungen vor. Es kann ggf. aber auch von Vorteil sein, wenn die Benzoxazol-Derivate in pigmentärer, d. h. ungelöster Form - beispielsweise in Partikelgrößen von 10 nm bis zu 300 nm - vorliegen.

Einige der öllöslichen UV-Filter können selbst als Lösungsmittel oder Lösungsvermittler für andere UV-Filter dienen. So lassen sich beispielsweise Lösungen des UV-A-Filters 1-(4-tert.-Butylphenyl)-3-(4'methoxyphenyl)propan-1,3-dion (z. B. Parsol^{®} 1789) in verschiedenen UV-B-Filtern herstellen. Die erfindungsgemäßen Zusammensetzungen enthalten daher in einer weiteren bevorzugten Ausführungsform 1-(4-tert.-Butylphenyl)-3-(4'-methoxyphenyl)propan-1,3-dion in Kombination mit mindestens einem UV-B-Filter, ausgewählt aus 4-Methoxyzimtsäure-2-ethylhexylester, 2-Cyano-3,3-phenylzimtsäure-2-ethylhexylester, Salicylsäure-2-ethylhexylester und 3,3,5-Trimethyl-cyclohexylsalicylat. In diesen Kombinationen liegt das Gewichtsverhältnis von UV-B-Filter zu 1-(4-tert.-Butylphenyl)-3-(4`methoxyphenyl)propan-1,3-dion zwischen 1:1 und 10:1, bevorzugt zwischen 2:1 und 8:1, das molare Verhältnis liegt entsprechend zwischen 0,3 und 3,8, bevorzugt zwischen 0,7 und 3,0.

Die Liste der genannten optionalen UV-Filtersubstanzen, die im Sinne der vorliegenden Erfindung eingesetzt werden können, soll selbstverständlich nicht limitierend sein.

Unabhängig davon, welche und wie viele UV-Filtersubstanzen im erfindungsgemäßen Verfahren eingesetzt werden, sind Verfahren bevorzugt, bei denen die Gesamtmenge an UV-Filtersubstanzen in der Emulsion - bezogen auf das Gesamtgewicht der Emulsion - 1 bis 25 Gew.-%, vorzugsweise 1,5 bis 22,5 Gew.-%, weiter bevorzugt 2 bis 20 Gew.-%, noch weiter bevorzugt 2,5 bis 15 Gew.-% und insbesondere 3 bis 10 Gew.-% beträgt.

Die nachfolgenden Beispiele sollen den Gegenstand der vorliegenden Anmeldung verdeutlichen, ohne ihn hierauf einzuschränken.

### Beispiele:

Die Zahlenwerte in den Beispielen bedeuten Gewichtsprozente, bezogen auf das Gesamtgewicht der fertig hergestellten Emulsionen.

Es wurden folgende Ölphasen hergestellt:

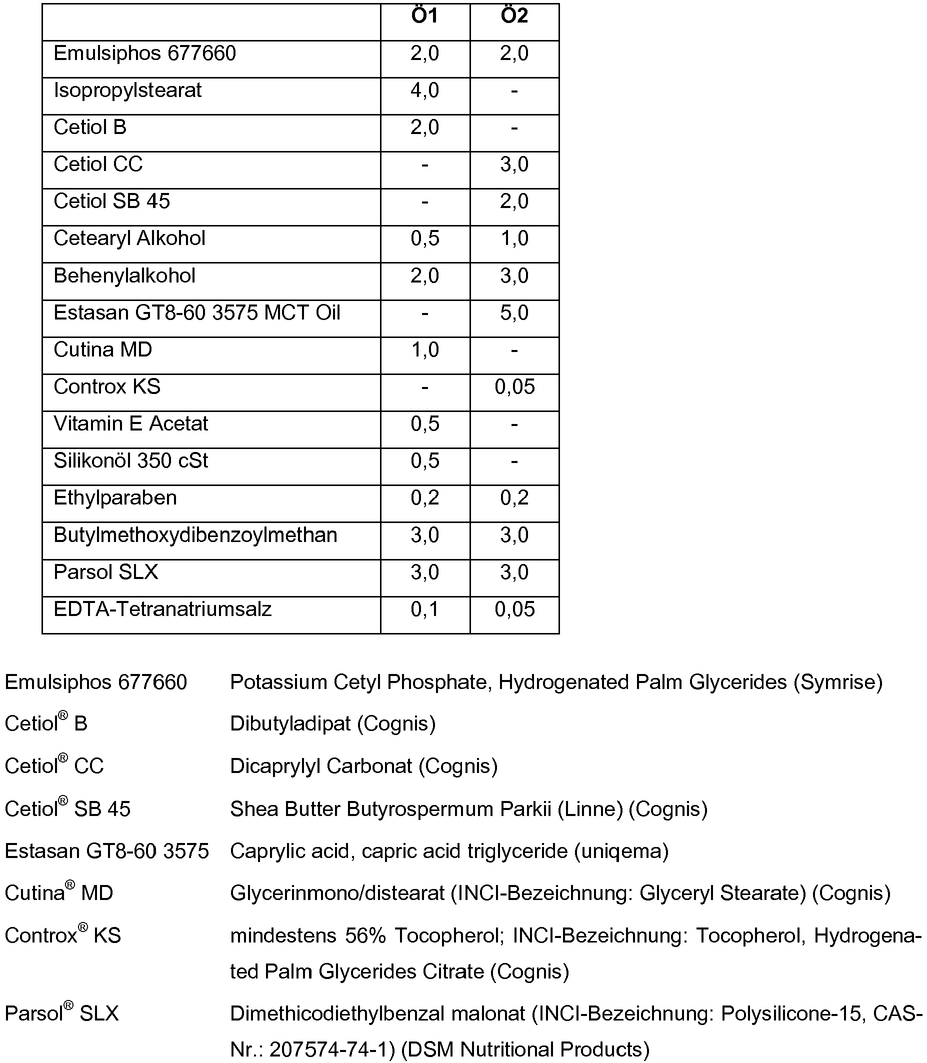

Weiter wurden folgende Wasserphasen hergestellt:

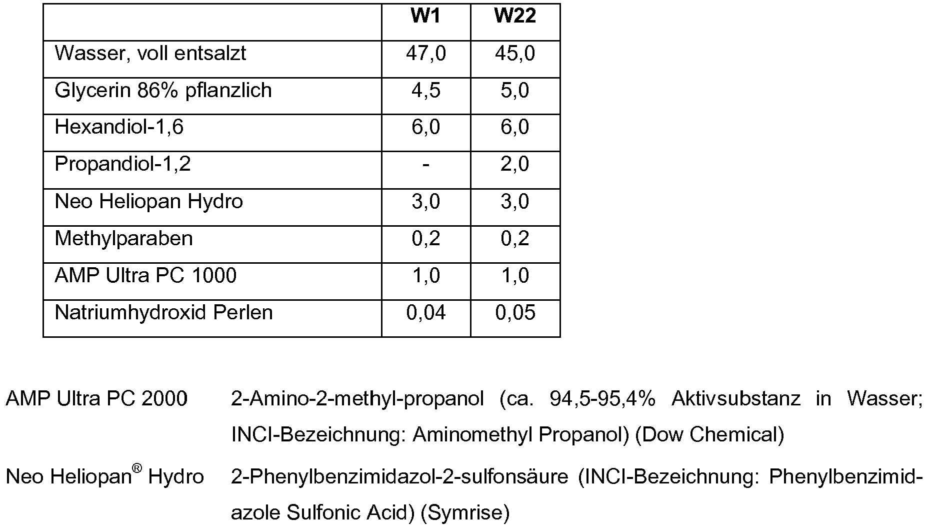

Die Ölphase und die Wasserphase wurden jeweils auf 85°C erhitzt, danach wurde die Wasserphase zur Ölphase gegeben und emulgiert. Im Anschluss an den Emulgiervorgang wurden weitere Inhaltsstoffe zugegeben:

| | | |
|---|---|---|
| Talkum | 1,0 | - |
| Titandioxid | 0,5 | - |
| Lipochroman-6 | 0,01 | 0,01 |
| Wasser, voll entsalzt | ad 100 | ad 100 |

Die Emulsionen waren lager- und farbstabil über mehrere Monate.

Emulsionen, bei denen das EDTA-Tetranatriumsalz nicht in die Ölphase gegeben wurde, sondern entweder über die Wasserphase oder über die später zugefügten Stoffe in die Emulsion eingebracht wurde, zeigten nach dreiwöchiger Lagerung inakzeptable Verfärbungen.

## Patentansprüche

1. Verfahren zur Herstellung von Emulsionen, welche Komplexbildner enthalten, **gekennzeichnet dadurch, dass** die Schritte:
a) Bereitstellen einer Ölphase, die - bezogen auf das Gesamtgewicht der Emulsion - 0,5 bis 20 Gew.-%, vorzugsweise 0,75 bis 15 Gew.-%, weiter bevorzugt 1 bis 15 Gew.-%, noch weiter bevorzugt 1,5 bis 10 Gew.-% und insbesondere 3 bis 8 Gew.-% UV-Filtersubstanzen enthält;
b) Einarbeitung mindestens eines Komplexbildners in die Ölphase, wobei der Komplexbildner in fester Form vorliegt;
c) Vermischen mit einer Wasserphase;
d) Emulgieren,
nacheinander durchgeführt werden, wobei die Ölphase mindestens einen öllöslichen UV-Filter, umfassend mindestens ein Alkyl- und/oder Alkoxy-substituiertes Dibenzoylmethanderivat und/oder Polysilicone-15, enthält und der Komplexbildner ausgewählt ist aus Ethylendiamintetraessigsäure (EDTA) und ihren Salzen, insbesondere Calcium-Dinatrium-EDTA, Nitrilotriessigsäure (NTA), Zitronensäure und ihren Salzen Natriumcitrat, Kaliumcitrat und Calciumcitrat, Phosphaten, Oxydisuccinaten, Ethylendiamin-N,N'-disuccinat (EDDS), bevorzugt in Form seiner Natrium- oder Magnesiumsalze, Glycerindisuccinaten, Glycerintrisuccinaten, Iminodisuccinat Tetranatriumsalz, Gluconsäure, insbesondere in Form des leicht kristallisierenden Glucono-δ-lactons, Isoascorbinsäure, Natriumisoascorbat und Weinsäure und ihren Salzen.

2. Verfahren nach Anspruch 1, **dadurch gekennzeichnet, dass** der Anteil der Ölphase 2 bis 50 Gew.-%, vorzugsweise 3 bis 45 Gew.-%, weiter bevorzugt 4 bis 40 Gew.-% und insbesondere 5 bis 30 Gew.-%, bezogen auf das Gesamtgewicht der Emulsion, beträgt.

3. Verfahren nach einem der Ansprüche 1 oder 2, **dadurch gekennzeichnet, dass** die Ölphase eine Temperatur von 60 bis 95°C, vorzugsweise von 65 bis 90°C, besonders bevorzugt von 70 bis 90°C und insbesondere von 75 bis 85°C aufweist.

4. Verfahren nach einem der Ansprüche 1 oder 2, **dadurch gekennzeichnet, dass** die Ölphase eine Temperatur von 15 bis 30°C, vorzugsweise von 18 bis 28°C, besonders bevorzugt von 20 bis 25°C, aufweist.

5. Verfahren nach einem der Ansprüche 1 bis 4, **dadurch gekennzeichnet, dass** der Anteil der Komplexbildner 0,01 bis 2,5 Gew.-%, vorzugsweise 0,025 bis 0,5 Gew.-%, weiter bevorzugt 0,05 bis 0,25 Gew.-% und insbesondere 0,06 bis 0,1 Gew.-%, bezogen auf das Gesamtgewicht der Emulsion, beträgt.

6. Verfahren nach einem der Ansprüche 1 bis 3 oder 5, **dadurch gekennzeichnet, dass** die Wasserphase eine Temperatur von 60 bis 95°C, vorzugsweise von 65 bis 90°C, besonders bevorzugt von 70 bis 90°C und insbesondere von 75 bis 85°C aufweist.

7. Verfahren nach einem der Ansprüche 1, 2, 4 oder 5, **dadurch gekennzeichnet, dass** die Wasserphase eine Temperatur von 15 bis 30°C, vorzugsweise von 18 bis 28°C, besonders bevorzugt von 20 bis 25°C, aufweist.

8. Verfahren nach einem der Ansprüche 1 bis 7, **dadurch gekennzeichnet, dass** die Wasserphase - bezogen auf das Gesamtgewicht der Emulsion - 0,5 bis 10 Gew.-%, vorzugsweise 0,75 bis 7,5 Gew.-%, weiter bevorzugt 1 bis 5 Gew.-%, noch weiter bevorzugt 1,5 bis 4 Gew.-% und insbesondere 2 bis 3 Gew.-% UV-Filtersubstanzen enthält.

9. Verfahren nach Anspruch 8, **dadurch gekennzeichnet, dass** die Wasserphase mindestens einen wasserlöslichen UV-Filter, umfassend ein Derivat der Benzimidazolsulfonsäure, enthält.

10. Verfahren nach einem der Ansprüche 1 bis 9, **dadurch gekennzeichnet, dass** die Gesamtmenge an UV-Filtersubstanzen in der Emulsion - bezogen auf das Gesamtgewicht der Emulsion - 1 bis 25 Gew.-%, vorzugsweise 1,5 bis 22,5 Gew.-%, weiter bevorzugt 2 bis 20 Gew.-%, noch weiter bevorzugt 2,5 bis 15 Gew.-% und insbesondere 3 bis 10 Gew.-% beträgt.

11. Verfahren nach einem der Ansprüche 1 bis 10, **dadurch gekennzeichnet, dass** es sich um eine Öl-in-Wasser-Emulsion handelt.

12. Verfahren nach einem der Ansprüche 1 bis 10, **dadurch gekennzeichnet, dass** es sich um eine Wasser-in-Öl-Emulsion handelt.

## Claims

1. Method for producing emulsions containing complex-forming agent, **characterized in that** the following steps of:
a) providing an oil phase which, based on the total weight of the emulsion, contains 0.5 to 20% by weight, preferably 0.75 to 15% by weight, more preferably 1 to 15% by weight, even more preferably 1.5 to 10% by weight, and in particular 3 to 8% by weight, of UV filter substances;
b) incorporating at least one complex-forming agent into the oil phase, the complex-forming agent being present in solid form;
c) admixing with a water phase;
d) emulsifying,
are carried out in succession, wherein the oil phase contains at least one oil-soluble UV filter comprising at least one alkyl- and/or alkoxy-substituted dibenzoylmethane derivative and/or Polysilicone-15, and the complex-forming agent is selected from ethylenediaminetetraacetic acid (EDTA) and the salts thereof, in particular calcium disodium EDTA; nitrilotriacetic acid (NTA), citric acid and its salts sodium citrate, potassium citrate, and calcium citrate; phosphates, oxydisuccinates, ethylenediamine *N,N'*-disuccinate (EDDS), preferably in the form of the sodium or magnesium salts thereof, glycerin disuccinates, glycerin trisuccinates, iminodisuccinate tetrasodium salt, gluconic acid, in particular in the form of the easily crystallized glucono-δ-lactone, isoascorbic acid, sodium isoascorbate, and tartaric acid and the salts thereof.

2. Method according to Claim 1, **characterized in that** the proportion of the oil phase is 2 to 50% by weight, preferably 3 to 45% by weight, more preferably 4 to 40% by weight, and in particular 5 to 30% by weight, based on the total weight of the emulsion.

3. Method according to one of Claims 1 or 2, **characterized in that** the oil phase has a temperature of 60 to 95°C, preferably 65 to 90°C, particularly preferably 70 to 90°C, and in particular 75 to 85°C.

4. Method according to one of Claims 1 or 2, **characterized in that** the oil phase has a temperature of 15 to 30°C, preferably 18 to 28°C, particularly preferably 20 to 25°C.

5. Method according to one of Claims 1 to 4, **characterized in that** the proportion of the complex-forming agents is 0.01 to 2.5% by weight, preferably 0.025 to 0.5% by weight, more preferably 0.05 to 0.25% by weight, and in particular 0.06 to 0.1% by weight, based on the total weight of the emulsion.

6. Method according to one of Claims 1 to 3 or 5, **characterized in that** the water phase has a temperature of 60 to 95°C, preferably 65 to 90°C, particularly preferably 70 to 90°C, and in particular 75 to 85°C.

7. Method according to one of Claims 1, 2, 4, or 5, **characterized in that** the water phase has a temperature of 15 to 30°C, preferably 18 to 28°C, particularly preferably 20 to 25°C.

8. Method according to one of Claims 1 to 7, **characterized in that** the water phase, based on the total weight of the emulsion, contains 0.5 to 10% by weight, preferably 0.75 to 7.5% by weight, more preferably 1 to 5% by weight, even more preferably 1.5 to 4% by weight, and in particular 2 to 3% by weight, of UV filter substances.

9. Method according to Claim 8, **characterized in that** the water phase contains at least one water-soluble UV filter comprising a derivative of benzimidazole sulfonic acid.

10. Method according to one of Claims 1 to 9, **characterized in that** the total quantity of UV filter substances in the emulsion, based on the total weight of the emulsion, is 1 to 25% by weight, preferably 1.5 to 22.5% by weight, more preferably 2 to 20% by weight, even more preferably 2.5 to 15% by weight, and in particular 3 to 10% by weight.

11. Method according to one of Claims 1 to 10, **characterized in that** the method involves an oil-in-water emulsion.

12. Method according to one of Claims 1 to 10, **characterized in that** the method involves a water-in-oil emulsion.

## Revendications

1. Procédé pour la préparation d'émulsions qui contiennent des agents complexants, **caractérisé en ce que** les étapes consistant à :
a) produire une phase huileuse qui contient - sur la base du poids total de l'émulsion - 0,5 à 20% en poids, de préférence de 0,75 à 15% en poids, de préférence 1 à 15% en poids, encore plus préférablement 1,5 à 10% en poids, et en particulier de 3 à 8% en poids de substances filtrant les UV ;
b) incorporer au moins un agent complexant dans la phase huileuse, l'agent complexant se présentant sous forme solide ;
c) mélanger avec une phase aqueuse ;
d) émulsionner,
sont effectuées séquentiellement, la phase huileuse contenant au moins un filtre UV soluble dans l'huile qui comprend au moins un dérivé du dibenzoylméthane et/ou polysilicone-15 avec un alkyle et/ou un alcoxy et l'agent complexant étant choisi parmi l'acide éthylènediaminetétraacétique (EDTA) et ses sels, en particulier le calcium-disodium-EDTA, l'acide nitrilotriacétique (NTA), l'acide citrique et ses sels, le citrate de sodium, le citrate de potassium et le citrate de calcium, les phosphates, les oxydisuccinates, l'éthylènediamine-N,N'-disuccinate (EDDS), de préférence sous la forme de ses sels de sodium ou dé magnésium, les disuccinates de glycérine. Les trisuccinaten de de glycérine, l'iminodisuccinate tétrasodiques, l'acide gluconique, en particulier sous la forme de glucono-δ-lactones faiblement cristallisées, l'acide isoascorbique, l'isoascorbate de sodium et l'acide tartrique et leurs sels.

2. Procédé selon la revendication 1, **caractérisé en ce que** la proportion de phase huileuse est de 2 à 50% en poids, de préférence de 3 à 45% en poids, de préférence de 4 à 40% en poids et en particulier de 5 à 30% en poids, sur la base du poids total de l'émulsion.

3. Procédé selon l'une des revendications 1 ou 2, **caractérisé en ce que** la phase huileuse a une température de 60 à 95°C, de préférence de 65 à 90°C, de manière particulièrement préférée de 70 à 90°C et en particulier de 75 à 85°C.

4. Procédé selon l'une des revendications 1 ou 2, **caractérisé en ce que** la phase huileuse a une température de 15 à 30°C, de préférence de 18 à 28°C, plus préférablement de 20 à 25°C.

5. Procédé selon l'une des revendications 1 à 4, **caractérisé en ce que** la proportion d'agent complexant est de 0,01 à 2,5% en poids, de préférence de 0,025 à 0,5% en poids, plus préférablement de 0,05 à 0,25% en poids et en particulier de 0,06 à 0,1% en poids, sur la base du poids total de l'émulsion.

6. Procédé selon l'une des revendications 1 à 3 ou 5, **caractérisé en ce que** la phase aqueuse a une température de 60 à 95°C, de préférence de 65 à 90°C, plus préférablement de 70 à 90°C et en particulier de 75 à 85°C.

7. Procédé selon l'une des revendications 1, 2, 4 ou 5, **caractérisé en ce que** la phase aqueuse a une température de 15 à 30°C, de préférence de 18 à 28°C, plus préférablement de 20 à 25°C.

8. Procédé selon l'une des revendications 1 à 7, **caractérisé en ce que** la phase aqueuse à, sur la base du poids total de l'émulsion, 0,5 à 10% en poids, de préférence de 0,75 à 7,5% en poids, plus préférablement 1 à 5% en poids, encore plus préférablement de 1,5 à 4% en poids et en particulier de 2 à 3% en poids de substances filtrant les UV.

9. Procédé selon la revendication 8, **caractérisé en ce que** la phase aqueuse comprend au moins un filtre UV hydrosoluble, comprenant un dérivé d'acide benzimidazolsulfonique.

10. Procédé selon l'une des revendications 1 à 9, **caractérisé en ce que** la quantité totale de substances filtrant les UV dans l'émulsion, sur la base du poids total de l'émulsion, est de 1 à 25% en poids, de préférence de 1,5 à 22,5% en poids, plus préférablement de 2 à 20% en poids, encore plus préférablement 2,5 à 15% en poids, et en particulier de 3 à 10% en poids.

11. Procédé selon l'une des revendications 1 à 10, **caractérisé en ce qu'**il s'agit d'une émulsion huile-dans-eau.

12. Procédé selon l'une des revendications 1 à 10, **caractérisé en ce qu'**il s'agit d'une émulsion eau-dans-huile.
